# EUROPÄISCHE PATENTANMELDUNG

(11) **EP 3 629 340 A1**
(43) Veröffentlichungstag der Anmeldung: **01.04.2020**
(21) Anmeldenummer: 19196841.1
(22) Anmeldetag: 12.09.2019
(51) Int. Cl.: G16H 40/63

(54) **MEDIZINISCHE BILDGEBUNGSVORRICHTUNG MIT EINER MEDIZINISCHEN SCANNEREINHEIT UND ZUMINDEST EINEM DISPLAY SOWIE EIN VERFAHREN ZU EINEM ANSTEUERN ZUMINDEST EINES DISPLAYS EINER MEDIZINISCHEN BILDGEBUNGSVORRICHTUNG**

(30) Priorität: 28.09.2018 EP 18197463
(71) Anmelder: Siemens Healthcare GmbH, 91052 Erlangen (DE)
(72) Erfinder: Karl, Harald, 90765 Fürth (DE); Höcht, Philipp, 91207 Lauf (DE); Wolf, Felix, 91052 Erlangen (DE)

(57) **Zusammenfassung**

Die Erfindung geht aus von einer medizinische Bildgebungsvorrichtung mit einer medizinischen Scannereinheit, einer Recheneinheit, die mit einer Master-Einheit verbunden ist, und zumindest einem Display, wobei das zumindest eine Display eine Slave-Einheit umfasst, wobei die Master-Einheit mittels einer Datenverbindung mit der Slave-Einheit verbunden ist

## Beschreibung

Die vorliegende Erfindung betrifft eine medizinische Bildgebungsvorrichtung mit einer medizinischen Scannereinheit, einer Recheneinheit, einer Master-Einheit, die mit der Recheneinheit verbunden ist, und zumindest einem Display. Des Weiteren geht die Erfindung aus von einem Verfahren zu einem Ansteuern zumindest eines Displays einer medizinischen Bildgebungsvorrichtung.

Medizinische Bildgebungsvorrichtungen weisen oft ein Display auf, das direkt an einer Scannereinheit der medizinischen Bildgebungsvorrichtung oder in einer Nähe der Scannereinheit angeordnet sein kann. Die Scannereinheit und damit das Display befinden sich innerhalb eines Untersuchungsraums. Ein Steuer-Computer, wie beispielsweise eine Recheneinheit, dagegen ist meist in einem Kontrollraum angeordnet. Das Display ist über eine Datenverbindung an den Steuer-Computer, insbesondere an die Recheneinheit, angebunden.

Ist die medizinische Bildgebungsvorrichtung beispielsweise von einer Magnetresonanzvorrichtung gebildet, muss zudem die Datenverbindung zwischen dem Display und dem Steuer-Computer über Glasfaser-Kabel erfolgen. Eine Verwendung von Kupferkabel ist aufgrund von elektrischen Feldern, die von den Kupferkabeln generiert werden können, problematisch, da diese elektrischen Felder eine Magnetresonanzbildgebung beeinflussen können.

Weist die medizinische Bildgebungsvorrichtung, insbesondere die Magnetresonanzvorrichtung, mehr als ein Display auf, muss bisher für jedes einzelne Display eine eigene Datenverbindung, insbesondere eine eigen Glasfaser-Verbindung, zum Steuer-Computer vorhanden sein. Die einzelnen Displays müssen hierfür auch einen Glasfaserumsetzer und/oder Glasfaserwandler aufweisen, so dass das Signal von dem Glasfaserkabel auf dem Display übertragen werden kann. Aktuell verfügbare Implementierung für eine Glasfaserübertragung sind jedoch nicht besonders robust, so dass dies zu Verbindungsproblemen zwischen den einzelnen Displays und dem Steuer-Computer, insbesondere der Recheneinheit, führen kann. Zudem sind derartige Glasfaserumsetzer und/oder Glasfaserwandler auch anfällig für Störungen bei einem häufigen Einschalten oder Ausschalten der einzelnen Displays. Zudem müssen häufig Displays, die an einem Steuer-Computer mittels eines Display-Port angeschlossen sind, nach jedem Einschalten zunächst erkannt werden und anschließend ein Link-Training durchgeführt werden, was wiederum sehr zeitaufwendig ist. Ein Ausschalten der einzelnen Displays erfolgt beispielsweise während einer Magnetresonanzuntersuchung, damit insbesondere eine Elektronik der Displays keine Störungen bei beispielsweise Magnetresonanzbilddaten verursacht.

Der vorliegenden Erfindung liegt insbesondere die Aufgabe zugrunde, eine sichere und robuste Datenübertragung zwischen einer Recheneinheit und einem innerhalb eines Untersuchungsraums angeordneten Display zu ermöglichen. Die Aufgabe wird durch die Merkmale der unabhängigen Ansprüche gelöst. Vorteilhafte Ausgestaltungen sind in den Unteransprüchen beschrieben.

Die Erfindung geht aus von einer medizinischen Bildgebungsvorrichtung mit einer medizinischen Scannereinheit, einer Recheneinheit, einer Master-Einheit, die mit der Recheneinheit verbunden ist, und zumindest einem Display. Dabei kann es vorgesehen sein, dass das zumindest eine Display eine Slave-Einheit umfasst, wobei die Master-Einheit mittels einer Datenverbindung der medizinischen Bildgebungsvorrichtung mit der Slave-Einheit verbunden ist.

Die medizinische Bildgebungsvorrichtung kann von allen, dem Fachmann als sinnvoll erscheinenden medizinischen Bildgebungsvorrichtungen gebildet sein, wie beispielsweise eine Röntgenvorrichtung, eine PET-Vorrichtung (Positron-Emissions-Tomographie-Vorrichtung) usw. Besonders vorteilhaft jedoch ist die medizinische Bildgebungsvorrichtung von einer Magnetresonanzvorrichtung gebildet, da hier aufgrund der Master-Slave-Beziehung eine einfache und sichere Datenübertragung zwischen der Recheneinheit und dem Display bereitgestellt werden kann. Zudem erlaubt es die Erfindung bei einer Magnetresonanzvorrichtung, das Display während der Magnetresonanzmessung auszuschalten und danach schnell wieder einzuschalten, um während der Messung keine elektromagnetischen-Artefakte zu erzeugen.

Die Scannereinheit der medizinischen Bildgebungsvorrichtung ist dazu ausgelegt, medizinische Bilddaten während einer medizinischen Bildgebungsuntersuchung zu erfassen. Die Scannereinheit ist hierzu bevorzugt in einem Untersuchungsraum angeordnet. Die Scannereinheit kann beispielsweise eine Magneteinheit oder auch einen Röntgendetektor usw. umfassen. Insbesondere ist auch das zumindest eine Display der medizinischen Bildgebungsvorrichtung innerhalb des Untersuchungsraums angeordnet. Die Recheneinheit ist zusammen mit der Master-Einheit dagegen in einem Kontrollraum angeordnet, wobei der Kontrollraum getrennt zu dem Untersuchungsraum ausgebildet ist. Insbesondere ist der Untersuchungsraum hinsichtlich eines Austauschs von elektromagnetischer Strahlung von dem Kontrollraum entkoppelt ausgebildet.

Die Recheneinheit ist zu einer Steuerung der medizinischen Bildgebungsvorrichtung ausgelegt. Hierzu weist die Recheneinheit eine erforderliche Software und/oder Computerprogramme auf, die in einem Speicher hinterlegt sind. Der Speicher kann dabei von der Recheneinheit umfasst sein oder auch separat zur Recheneinheit ausgebildet sein. Dabei kann der Speicher auch von der medizinischen Bildgebungsvorrichtung umfasst sein oder von einem externen Speicher gebildet sein. Die Computerprogramme und/oder Software können in einem Prozessor der Recheneinheit ausgeführt werden, wobei bei einer Ausführung der Computerprogramme und/oder Software eine Steuerung der medizinischen Bildgebungsvorrichtung erfolgt.

Das zumindest eine Display umfasst bevorzugt einen Monitor und/oder ein Touch-Display und ist innerhalb des Untersuchungsraums angeordnet sein. Dabei kann das zumindest eine Display direkt an der Scannereinheit angeordnet sein, wie beispielsweise an einer Frontseite der Scannereinheit und/oder an einer Patientenliege der Scannereinheit. Die medizinische Bildgebungsvorrichtung kann dabei ein einziges Display oder auch zwei oder mehr Displays aufweisen, die innerhalb des Untersuchungsraums angeordnet sind. Bevorzugt weist jedes der Displays eine eigene Slave-Einheit für eine Kommunikation mit der Master-Einheit auf. Zudem kann das zumindest eine Display, das bevorzugt einen Monitor und/oder ein Touch-Display umfasst, auch innerhalb eines Bedienraums angeordnet sein. Insbesondere sind das zumindest eine Display und die Master-Einheit in unterschiedlichen Räumen angeordnet.

Die Master-Einheit umfasst bevorzugt eine Einheit, mittels der Zugriffe auf eine Ressource geregelt werden können. Die Master-Einheit weist hierzu bevorzugt eine Logikeinheit und/oder eine logische Schaltung auf, wie beispielsweise ein FPGA (Field Programmable Gate Array), die einzelne Zugriffsrechte regelt. Die Logikeinheit und/oder die logische Schaltung regelt dabei die Zugriffsrechte für insbesondere die Slave-Einheit des zumindest einen Displays auf einen Zugriff auf die übertragenen Daten. Bevorzugt weist hierbei die Logikeinheit und/oder die logische Schaltung ein programmierbares und/oder konfigurierbares FPGA auf. Besonders bevorzugt weist die Logikeinheit und/oder die logische Schaltung ein mehrmals programmierbares und/oder mehrmals konfigurierbares FPGA auf. Vorzugsweise wird in der Logikeinheit und/oder der logischen Schaltung der Master-Einheit das Übertragungsprotokoll für die Slave-Einheit des zumindest einen Displays generiert. Das Übertragungsprotokoll umfasst Daten, die zur Ansteuerung der einzelnen Displays und zur Darstellung von Darstellungsdaten mittels der einzelnen Displays vorgesehen sind. Die Logikeinheit, insbesondere das FPGA, umfasst beispielsweise ein SerDes (Serialisierer/Deserialisierer), die eine serielle Schnittstelle umfasst.

Die Master-Einheit kann dabei eine separat zur Recheneinheit ausgebildete Einheit umfassen. Alternativ hierzu kann die Master-Einheit auch von der Recheneinheit umfasst sein oder auch in die Recheneinheit integriert sein.

Die Slave-Einheit des zumindest einen Displays dagegen empfängt ihre Zugriffsrechte von der Master-Einheit. Die Slave-Einheit des zumindest einen Displays weist hierzu bevorzugt ebenfalls eine Logikeinheit und/oder eine logische Schaltung, wie beispielsweise ein FPGA, auf, um das empfangene Protokoll, insbesondere das empfangene Übertragungsprotokoll, innerhalb des Displays korrekt auszuführen und/oder umzusetzen. Vorzugsweise werden bei der Generierung des Übertragungsprotokolls innerhalb der Master-Einheit, insbesondere innerhalb der Logikeinheit, bereits Standard-Bilddaten in das Übertragungsprotokoll übertragen.

Die Datenverbindung umfasst vorzugsweise eine Verbindung zwischen der Master-Einheit und der Slave-Einheit des zumindest einen Displays. Mittels der Datenverbindung kann bevorzugt das Übertragungsprotokoll, insbesondere ein HMINet-Protokoll (Human Machine Interface Net-Protokoll), von der Master-Einheit auf die Slave-Einheit des zumindest einen Displays und/oder von der Slave-Einheit des zumindest einen Displays auf die Master-Einheit übertragen werden. Die Datenverbindung kann von allen, dem Fachmann als sinnvoll erscheinenden Datenverbindungen gebildet sein.

Durch die erfindungsgemäße Ausgestaltung kann vorteilhaft auf Standardschnittstellen mit Signalwandlern bei der Datenübertragung von der Recheneinheit zu zumindest einem Display innerhalb des Untersuchungsraums verzichtet werden. Derartige Standardschnittstellen können beispielsweise eine Digital Visual Interface (DVI) und/oder eine Display-Port-Schnittstelle und/oder eine Universal Serial Bus Schnittstelle (USB-Schnittstelle) umfassen. Derartige Standardschnittstellen mit den dazugehörigen Signalwandlern, beispielsweise Signalwandler zur Wandlung eines elektrischen Signals in ein Glasfasersignal und zurück, sind derzeit anfällig für Störungen und können somit zu einer Behinderung und/oder Beeinträchtigung einer Datenübertragen führen. Aufgrund der vorliegenden Erfindung kann dagegen eine robuste Ausgestaltung einer Verbindung zwischen der Recheneinheit, insbesondere der Master-Einheit, und einem Display, das innerhalb des Untersuchungsbereichs angeordnet ist, erfolgen. Vorzugsweise erfolgt hierbei eine Anbindung der Datenverbindung direkt an der Master-Einheit und/oder an der Slave-Einheit, so dass auf zusätzliche Signalwandler verzichtet werden kann. Zudem können hierbei unterschiedliche Standard-Bildsignale und/oder Standard-Bilddaten und/oder Steuerdaten zur Ansteuerung des zumindest einen Displays mittels einer Datenverbindung zwischen der Master-Einheit und der Slave-Einheit ausgetauscht werden.

Zudem kann durch die Verwendung eines HMINet-Protokolls ein Display-Port nur im HMINet der Master-Einheit terminiert werden, d.h. aus Sicht eines Host-PC, der eine Benutzerschnittstelle umfasst, ist das Display immer an. Das Ausschalten und/oder Abschalten des Displays erfährt nur die Master-Einheit bzw. bekommt nur die Master-Einheit mit. Die Verbindung Master-Einheit zu Slave-Einheit ist dahingehend optimiert, dass ein schnelles Einschalten möglich ist. Dies wird dadurch erreicht, dass Pixel-Daten, die das Display ansteuern, direkt übertragen werden und sich die Verbindung sehr schnell wieder aufbaut.

In einer vorteilhaften Weiterbildung der medizinischen Bildgebungsvorrichtung kann es vorgesehen sein, dass die Datenverbindung eine optische Datenverbindung zwischen der Slave-Einheit und der Master-Einheit umfasst. Die optische Datenverbindung kann beispielsweise Lichtwellenleiter oder Glasfaserkabel umfassen. Zudem kann die optische Datenverbindung eine optische Hochgeschwindigkeits-Datenverbindung, wie beispielsweise mittels Hochgeschwindigkeits-Glasfasern, umfassen. Hierdurch kann bei insbesondere Magnetresonanzvorrichtungen ein störungsfreier Betrieb, insbesondere ein störungsfreies Erfassen von Magnetresonanzbilddaten, erreicht werden. Zudem kann ein Verwenden von Kupferleitungen und/oder elektrischen Leitungen innerhalb des Untersuchungsraums vorteilhaft verhindert werden und damit auch eine Beeinträchtigung einer Magnetresonanzuntersuchung durch die Kupferleitungen und/oder die elektrischen Leitungen verhindert werden.

In einer vorteilhaften Weiterbildung der medizinischen Bildgebungsvorrichtung kann es vorgesehen sein, dass die Datenverbindung eine bidirektionale Datenverbindung umfasst. Hierdurch kann sowohl eine Datenübertragung von der Master-Einheit auf die Slave-Einheit des zumindest einen Displays und/oder von der Slave-Einheit des zumindest einen Displays auf die Master-Einheit besonders einfach erfolgen.

In einer vorteilhaften Weiterbildung der medizinischen Bildgebungsvorrichtung kann es vorgesehen sein, dass die Master-Einheit eine Logikeinheit aufweist, mittels der ein Übertragungsprotokoll generierbar ist. Bei der Generierung des Übertragungsprotokolls können beispielsweise Standard-Bildsignale umgewandelt werden. Standard-Bildsignale können beispielsweise Bildsignale umfassen, die mittels USB (Universal Serial Bus), insbesondere USB2 oder USB3, und/oder mittels I²C (Inter-Integrated Circuit) und/oder mittels Display-Port und/oder LVDS (Low Voltage Differential Signaling) übertragen werden können. Die Logikeinheit und/oder die logische Schaltung umfasst bevorzugt ein programmierbares und/oder konfigurierbares FPGA. Besonders bevorzugt weist die Logikeinheit und/oder die logische Schaltung ein mehrmals programmierbares und/oder mehrmals konfigurierbares FPGA auf, wobei das FPGA derart konfiguriert und/oder programmiert ist, dass Standard-Bildsignale und/oder Steuersignale für das zumindest eine Display vor der Datenübertragung an die Slave-Einheit umgewandelt werden. Derart können bereits innerhalb der Master-Einheit die entsprechenden Signale umgewandelt werden. Dies ermöglicht eine insbesondere robuste und/oder stabile Datenverbindung für eine Datenübertragung zwischen der Master-Einheit und der Slave-Einheit. Zudem kann derart auf zusätzliche Signalwandler vorteilhaft verzichtet werden.

Insbesondere umfassen die Baueinheiten und/oder Bauteile der Master-Einheit programmierbare Logikeinheiten, insbesondere reprogrammierbare Logikeinheiten, so dass eine einfache Anpassung an neue Standards und/oder Anforderungen durch eine neue Programmierung der Logikeinheiten erfolgen kann. Hierdurch kann auch ein Austausch der Einheiten, insbesondere der Hardware-Einheiten, vorteilhaft verhindert werden. Zudem können die Logikeinheiten auch universell programmierbare Logikeinheiten umfassen, was einen Austausch von Logikeinheiten vereinfacht. Hierdurch kann ein universell programmierbarer Baustein, insbesondere die Logikeinheit, einfach gegen einen anderen universell programmierbaren Baustein, insbesondere die Logikeinheit, der Master-Einheit ausgetauscht werden.

In einer vorteilhaften Weiterbildung der medizinischen Bildgebungsvorrichtung kann es vorgesehen sein, dass die zumindest eine Slave-Einheit eine Logikeinheit umfasst, mittels der ein Übertragungsprotokoll ausführbar ist. Die Logikeinheit umfasst bevorzugt ein FPGA, der derart konfiguriert und/oder programmiert ist, dass das Übertragungsprotokoll, insbesondere das HMINet-Protokoll, in Steuersignale für das zumindest eine Display und/oder in Standard-Bildsignale für Displayschnittstellen, wie beispielsweise LVDS und/oder I²C und/oder USB, übertragen wird. Beispielsweise kann die LVDS-Schnittstelle für LVDS-Daten und/oder LVDS-Signale zu einer Ausgabe an einem LCD-Panel und/oder eine Ansteuerung eines LCD-Panels des zumindest einen Displays verwendet werden. Die I²C-Schnittstelle kann beispielsweise für I²C-Daten und/oder die I²C-Signale für eine Ausgabe an einem Touch-Controller und/oder eine Ansteuerung eines Touch-Controllers des zumindest einen Displays verwendet werden usw. Diese Ausgestaltung der Erfindung ermöglicht eine einfache Ausführung des Übertragungsprotokolls und damit eine einfache Bereitstellung von Darstellungsdaten und/oder Steuerdaten für das zumindest eine Display. Zudem kann derart auch auf zusätzliche Signalwandler vorteilhaft verzichtet werden.

Insbesondere umfassen die Baueinheiten der Slave-Einheit programmierbare Logikeinheiten, insbesondere reprogrammierbare Logikeinheiten, so dass eine einfache Anpassung an neue Standards und/oder Anforderungen durch eine neue Programmierung der Logikeinheiten erfolgen kann. Hierdurch kann auch ein Austausch der Einheiten, insbesondere der Hardware-Einheiten, vorteilhaft verhindert werden. Zudem können die Logikeinheiten auch universell programmierbare Logikeinheiten umfassen, was einen Austausch von Logikeinheiten vereinfacht. Hierdurch kann ein universell programmierbarer Baustein, insbesondere die Logikeinheit, einfach gegen einen anderen universell programmierbaren Baustein, insbesondere die Logikeinheit, der Slave-Einheit ausgetauscht werden.

In einer vorteilhaften Weiterbildung der medizinischen Bildgebungsvorrichtung kann es vorgesehen sein, dass die Slave-Einheit des zumindest einen Displays eine Schnittstelle zur Weitergabe der empfangenen Steuersignale und/oder Darstellungsdaten umfasst. Mittels der Schnittstelle und/oder eines Interfaces können beispielsweise Steuersignale und/oder Darstellungsdaten über eine LVDS-Schnittstelle an ein LCD-Panel des zumindest einen Displays weitergegeben und/oder weitergeleitet werden. Zudem können mittels der Schnittstelle und/oder des Interfaces beispielsweise Daten über eine I²C-Schnittstelle an ein Touch-Panel und/oder einen Touch-Controller des Displays weitergegeben und/oder weitergeleitet werden.

In einer vorteilhaften Weiterbildung der medizinischen Bildgebungsvorrichtung kann es vorgesehen sein, dass das zumindest eine Display eine Transceiver-Einheit und die Master-Einheit eine Transceiver-Einheit aufweist. Die Transceiver-Einheit ist bevorzugt zu einem Senden und/oder Empfangen von Daten und/oder Signalen ausgebildet. Vorzugsweise ist die Datenverbindung zwischen der Master-Einheit und dem Display zwischen der Transceiver-Einheit der Master-Einheit und der Transceiver-Einheit des zumindest einen Displays angeordnet. Die Transceiver-Einheit des Displays ist bevorzugt direkt mit der Slave-Einheit des Displays gekoppelt und/oder von der Slave-Einheit umfasst. Vorteilhafterweise umfasst die Transceiver-Einheit der Master-Einheit und/oder die Transceiver-Einheit des Displays eine Faseroptik-Transceiver-Einheit. Hierdurch kann eine vorteilhafte Übertragung eine Übertragungsprotokolls, insbesondere eines HMINet-Protokolls, zwischen der Master-Einheit und dem zumindest einem Display, das innerhalb des Untersuchungsraums angeordnet ist, erfolgen.

In einer vorteilhaften Weiterbildung der medizinischen Bildgebungsvorrichtung kann es vorgesehen sein, dass das Display von einem ersten Display gebildet ist, wobei die medizinische Bildgebungsvorrichtung zumindest ein weiteres Display mit einer Slave-Einheit umfasst. Die medizinische Bildgebungsvorrichtung kann dabei ein einziges weiteres Display oder auch zwei oder mehr weitere Displays umfassen. Bevorzugt umfasst dabei jedes der weiteren Displays eine eigene Slave-Einheit mit einer Logikeinheit, insbesondere einem FPGA. Derart kann eine einfache Datenübertragung zwischen der Recheneinheit und den einzelnen Displays erfolgen. Insbesondere kann derart auch eine einfache Zuordnung von Steuerdaten und/oder Darstellungsdaten für das jeweilige Display mittels der Master-Einheit erfolgen.

Die Logikeinheit der Master-Einheit, beispielsweise das FPGA, kann dabei derart ausgebildet sein, dass mittels eines einzigen FPGAs ein Übertragungsprotokoll von bis zu einer Anzahl von vier Displays der medizinischen Bildgebungsvorrichtung generiert werden kann. Auch kann das Übertragungsprotokoll für mehrere Displays mittels einer Datenverbindung, insbesondere einer einzigen Datenverbindung, an die Displays übertragen werden. Umfasst die medizinische Bildgebungsvorrichtung mehr als vier Displays, kann es vorgesehen sein, dass mehr als ein einziges FPGA innerhalb der Logikeinheit angeordnet ist oder auch dass die Logikeinheit weitere Einheiten aufweist, die ein Übertragungsprotokoll für mehr als vier Displays der medizinischen Bildgebungsvorrichtung generieren können.

In einer vorteilhaften Weiterbildung der medizinischen Bildgebungsvorrichtung kann es vorgesehen sein, dass die medizinische Bildgebungsvorrichtung n Displays aufweist, wobei n≥2 ist und wobei das (n-1)-te Display eine Transceiver-Einheit für eine Datenverbindung mit dem n-ten Display aufweist. Hierbei umfasst bevorzugt das Übertragungsprotokoll Informationen zu den Steuerdaten und/oder Darstellungsdaten des n-ten Displays und auch zu den Steuerdaten und/oder Darstellungsdaten des (n-1)-ten Displays. Insbesondere können hierbei mittels des Übertragungsprotokolls die Steuerdaten und/oder Darstellungsdaten für das n-ten Display an dem (n-1)-ten Display durchgeschleift werden. Vorteilhafterweise können hierbei Datenverbindungen zwischen der Master-Einheit und den einzelnen Displays und damit auch zwischen dem Kontrollraum und den Untersuchungsraum auf ein Minimum reduziert werden.

In einer vorteilhaften Weiterbildung der medizinischen Bildgebungsvorrichtung kann es vorgesehen sein, dass jedes (n-1)-te Display eine erste Transceiver-Einheit für einen Eingang des Übertragungsprotokolls und eine zweite Transceiver-Einheit für ein Weiterleiten des Übertragungsprotokolls aufweist. Vorzugsweise ist die erste Transceiver-Einheit des ersten Displays für einen Eingang und/oder Empfang des Übertragungsprotokolls direkt von der Master-Einheit oder von dem (n-2)-ten Display vorgesehen. Die zweite Transceiver-Einheit ist bevorzugt für ein Weiterleiten des Übertragungsprotokolls an ein weitere Display, insbesondere das n-te Display, vorgesehen. Insbesondere können derart mittels des Übertragungsprotokolls die Steuerdaten und/oder Darstellungsdaten für mehrere Displays mittels einer Datenverbindung, insbesondere einer einzigen Datenverbindung, übertragen werden und an den davor geschalteten Displays durchgeschleift werden, bis das Übertragungsprotokoll am Bestimmungsort, insbesondere an einem der n Displays, angekommen ist. Vorteilhafterweise können hierbei ebenfalls Datenverbindungen zwischen der Master-Einheit und den einzelnen Displays und damit auch zwischen dem Kontrollraum und den Untersuchungsraum auf ein Minimum reduziert werden.

In einer vorteilhaften Weiterbildung der medizinischen Bildgebungsvorrichtung kann es vorgesehen sein, dass ein Betriebsmodus des n-ten Displays zumindest teilweise abhängig ist von einem Betriebsmodus des (n-1)-ten Displays. Hierbei soll unter einem Betriebsmodus eines Displays insbesondere ein inaktiver Betriebsmodus und/oder Betriebszustand des Displays oder auch ein aktiver Betriebsmodus und/oder Betriebszustand des Displays verstanden werden. Der inaktive Zustand kann beispielsweise einen ausgeschalteten Zustand des Displays oder auch einen Bereitschaftszustand des Displays umfassen. Der aktive Zustand umfasst insbesondere einen Zustand, bei dem das Display in einem eingeschalteten Zustand ist und Daten anzeigt. Bei insbesondere medizinischen Bildgebungsuntersuchungen, die von einer Magnetresonanzuntersuchung gebildet sind, ist es vorteilhaft, dass während der medizinischen Bildgebungsuntersuchung die einzelnen Displays innerhalb des Untersuchungsraums ausgeschaltet werden, um die medizinische Bildgebungsuntersuchung, insbesondere die Magnetresonanzuntersuchung, nicht zu beeinträchtigen oder zu stören. Somit kann ein einfaches Ausschalten der Displays oder auch ein einfaches und schnelles inaktivieren der Displays innerhalb des Untersuchungsraums erreicht werden, da beispielsweise nur das erste Display entlang der Datenübertragungskette inaktiviert oder ausgeschaltet werden muss, um auch die weiteren Displays zu inaktivieren.

Vorzugsweise erfolgt ein Inaktivieren und/oder ein Deaktivieren, wie beispielsweise ein Abschalten, der einzelnen Displays automatisch mittels der Recheneinheit, wie beispielsweise während einer medizinischen Bildgebungsuntersuchung, so dass eine Störung der medizinischen Bildgebungsuntersuchung, insbesondere eine Erfassung von Magnetresonanzbilddaten, durch die Displays vorteilhaft verhindert werden kann. Das Deaktivieren oder Abschalten der einzelnen Displays kann hierbei von der Master-Einheit erfasst werden.

In einer vorteilhaften Weiterbildung der medizinischen Bildgebungsvorrichtung kann es vorgesehen sein, dass die Master-Einheit eine erste Transceiver Einheit umfasst für eine Übertragung eines Übertragungsprotokolls an das zumindest eine Display und eine zweite Transceiver-Einheit für eine Übertragung eines weiteren Übertragungsprotokolls an eine weitere Einheit, die eine Slave-Einheit umfasst. Die weitere Einheit kann eine beliebige, dem Fachmann als sinnvoll erscheinende Einheit umfassen. Besonders vorteilhaft kann die weitere Einheit ein weiteres Display umfassen. Beispielsweise können derart auch zwei unterschiedliche Übertragungsprotokolle für eine Darstellung mittels der unterschiedlichen Displays innerhalb des Untersuchungsraums zur Verfügung stehen. Zudem kann die weitere Einheit auch eine USB-Schnittstelle für eine Anbindung eines USB-geeigneten Geräts umfassen.

In einer vorteilhaften Weiterbildung der medizinischen Bildgebungsvorrichtung kann es vorgesehen sein, dass die Master-Einheit ein Ethernet-Modul umfasst. Mittels des Ethernet-Moduls kann ein einfacher und schneller Zugang zu der Logikeinheit, insbesondere dem FPGA, für einen Benutzer erfolgen. Beispielsweise kann mittels des Ethernet-Moduls eine Ethernet-basierte Diagnose der Master-Einheit, insbesondere des FPGA der Master-Einheit, erfolgen. Zudem können auch Updates für die einzelnen Einheiten der Master-Einheit oder auch Updates für die einzelnen Displays oder Slave-Einheiten aktualisiert werden. Das Ethernet-Modul kann beispielsweise eine Ethernet-Verbindung zwischen beispielsweise einem Micro-Controller, der innerhalb der Master-Einheit für beispielsweise eine Konfigurierung und/oder Implementierung der Logikeinheit angeordnet ist, zu der Logikeinheit, insbesondere dem FPGA, und zu einer USB-Schnittstelle aufweisen. Mittels der USB-Schnittstelle kann beispielsweise eine Verbindung zwischen der Master-Einheit und einem Host-PC, der bevorzugt eine Benutzerschnittstelle umfasst, hergestellt werden. Die USB-Schnittstelle kann dabei eine USB2-Schnittstelle oder auch eine USB3-Schnittstelle umfassen.

In einer vorteilhaften Weiterbildung der medizinischen Bildgebungsvorrichtung kann es vorgesehen sein, dass die medizinische Bildgebungsvorrichtung einen zentralen Host-PC aufweist, der zusammen mit der Master-Einheit innerhalb eines Kontrollraums angeordnet ist. Der zentrale Host-PC umfasst bevorzugt eine Benutzerschnittstelle mit einer Eingabeeinheit und einer Ausgabeeinheit. Der zentrale Host-PC kann vorteilhaft von der Recheneinheit umfasst sein. Mittels des zentralen Host-PCs kann eine Konfigurierung und/oder Implementierung der Master-Einheit durch einen Benutzer erfolgen oder auch eine Konfigurierung und/oder Implementierung der Master-Einheit von einem Benutzer kontrolliert werden. Zudem kann mittels des zentralen Host-PCs eine Konfigurierung und/oder Implementierung des Übertragungsprotokolls durch einen Benutzer erfolgen oder auch eine Konfigurierung und/oder Implementierung des Übertragungsprotokolls von einem Benutzer kontrolliert werden. Des Weiteren kann mittels des zentralen Host-PCs eine Verbindungsdiagnose zwischen der Master-Einheit und den Displays und/oder zwischen den einzelnen Displays erstellt werden und/oder einem Benutzer angezeigt werden. Insbesondere kann derart auch ein Netzwerkmanagement für die Master-Einheit ermöglicht werden.

In einer vorteilhaften Weiterbildung der medizinischen Bildgebungsvorrichtung kann es vorgesehen sein, dass die Master-Einheit zumindest eine standardisierte Datenschnittstelle und/oder Grafikschnittstelle aufweist zu einem Datenaustausch mit dem zentralen Host-PC. Hierdurch kann vorteilhaft eine direkte Verbindung zwischen der Master-Einheit und dem Host-PC bereitgestellt werden. Dies ermöglicht auch eine einfache Konfigurierung und/oder Implementierung der Master-Einheit und/oder des Übertragungsprotokolls.

Die vorliegende Erfindung ermöglicht es, dass ein universeller Schnittstellen-Konverter zur Verfügung zu stellen, um die Slave-Einheit bidirektional an die Master-Einheit zu koppeln, jedoch die Slave-Einheit von dem Host-PC, der die Benutzerschnittstelle umfasst, zu entkoppeln. Der universelle Schnittstellen-Konverter kann dabei Standard-Schnittstellen, wie beispielsweise Display-Port, USB, Ethernet und/oder Thunderbolt. Der Host-PC ist nur mit der Master-Einheit verbunden. Dadurch können Eingabedaten am Display, wie insbesondere Touchdaten, wie beispielsweise in Form von I2C-Daten und/oder SPI-Daten, über die Slave-Einheit an die Master-Einheit, insbesondere an einen Microkontroller der Master-Einheit, übertragen werden und dort einfach auf USB umgesetzt werden. Die umgesetzten Daten können dann an den Host-PC über ein Standard-USB-Interface weitergegeben werden. Der Host-PC erhält bevorzugt Daten immer über das Standard-USB-Interface von der Master-Einheit.

Des Weiteren geht die Erfindung von einem Verfahren zu einem Ansteuern zumindest eines Displays einer medizinischen Bildgebungsvorrichtung, mit den folgenden Schritten:
- Bereitstellen eines Übertragungsprotokolls innerhalb der Master-Einheit,
- Übertragen des Übertragungsprotokolls mittels der Datenverbindung an die Slave-Einheit des zumindest einen Displays und
- Ausführen des Übertragungsprotokolls in der Slave-Einheit.

Die medizinische Bildgebungsvorrichtung umfasst bevorzugt eine medizinische Scannereinheit, eine Recheneinheit, eine Master-Einheit, die mit der Recheneinheit verbunden ist, und zumindest ein Display. Dabei kann es vorgesehen sein, dass das zumindest eine Display eine Slave-Einheit umfasst, wobei die Master-Einheit mittels einer Datenverbindung der medizinischen Bildgebungsvorrichtung mit der Slave-Einheit verbunden ist. Die Master-Einheit kann dabei eine separat zur Recheneinheit ausgebildete Einheit umfassen. Alternativ hierzu kann die Master-Einheit auch von der Recheneinheit umfasst sein oder auch in die Recheneinheit integriert sein.

Mittels des erfindungsgemäßen Verfahrens kann vorteilhaft auf Standardschnittstellen mit Signalwandlern bei der Datenübertragung von der Recheneinheit zu zumindest einem Display innerhalb des Untersuchungsraums verzichtet werden. Insbesondere kann mittels der vorliegenden Erfindung eine robuste Ausgestaltung einer Verbindung zwischen der Recheneinheit, insbesondere der Master-Einheit, und einem Display, das innerhalb des Untersuchungsbereichs angeordnet ist, erfolgen. Vorzugsweise erfolgt hierbei eine Anbindung der Datenverbindung direkt an der Master-Einheit und/oder an der Slave-Einheit, so dass auf zusätzliche Signalwandler verzichtet werden kann. Zudem können hierbei unterschiedliche Standard-Bildsignale und/oder Standard-Bilddaten und/oder Steuerdaten zur Ansteuerung des zumindest einen Displays mittels einer Datenverbindung zwischen der Master-Einheit und der Slave-Einheit ausgetauscht werden.

Die Vorteile des erfindungsgemäßen Verfahrens zu einem Ansteuern zumindest eines Displays einer medizinischen Bildgebungsvorrichtung entsprechen im Wesentlichen den Vorteilen der erfindungsgemäßen medizinischen Bildgebungsvorrichtung, welche vorab im Detail ausgeführt sind. Hierbei erwähnte Merkmale, Vorteile oder alternative Ausführungsformen können ebenso auch auf die anderen beanspruchten Gegenstände übertragen werden und umgekehrt.

In einer vorteilhaften Weiterbildung des erfindungsgemäßen Verfahrens kann es vorgesehen sein, dass das Übertragungsprotokoll Daten für ein erstes Display und Daten für zumindest ein weiteres Display umfasst, wobei die Daten für das zumindest eine weitere Display von der Master-Einheit an die Slave-Einheit des ersten Displays übertragen werden und von der Slave-Einheit des ersten Displays an eine Slave-Einheit des zumindest einen weiteren Displays übertragen werden. Die Daten des Übertragungsprotokolls umfassen insbesondere Steuerdaten und/oder Darstellungsdaten für das erste Display und Steuerdaten und/oder Darstellungsdaten für das zumindest eine weitere Display. Insbesondere können hierbei mittels des Übertragungsprotokolls Steuerdaten und/oder Darstellungsdaten für das zumindest eine weitere Display an dem ersten Display durchgeschleift werden. Zudem kann derart mittels einer Datenverbindung, insbesondere einer einzigen Datenverbindung, zwischen der Master-Einheit und des Displays das Übertragungsprotokoll, insbesondere Steuerdaten und Darstellungsdaten, für mehrere Displays übertragen und/oder übermittelt werden. Dabei wird das Übertragungsprotokoll, insbesondere Steuerdaten und Darstellungsdaten, für ein weiteres Display an den davor geschalteten Displays durchgeschleift, bis das Übertragungsprotokoll am Bestimmungsort, insbesondere an einem der weiteren Displays, angekommen ist. Vorteilhafterweise können hierbei Datenverbindungen zwischen der Master-Einheit und den einzelnen Displays und damit auch zwischen dem Kontrollraum und den Untersuchungsraum auf ein Minimum reduziert werden und damit Bauteile und Kosten vorteilhaft eingespart werden.

In einer vorteilhaften Weiterbildung des erfindungsgemäßen Verfahrens kann es vorgesehen sein, dass eine Ethernet-basierte Auswertung einer Verbindung zwischen der Master-Einheit und dem zumindest einen Display und/oder ein Ethernet-basiertes Netzwerkmanagement durchgeführt wird. Insbesondere kann derart ein einfacher Zugang und/oder Zugriff zur Master-Einheit, insbesondere deiner Logikeinheit der Master-Einheit, erfolgen. Vorzugsweise erfolgt der Zugriff zusammen mit einem Ethernet-Modul der Master-Einheit und einem Host-PC, der eine Benutzerschnittstelle aufweist.

In einer vorteilhaften Weiterbildung des erfindungsgemäßen Verfahrens kann es vorgesehen sein, dass ein automatisches Abschalten des zumindest einen Displays während einer medizinischen Bildgebungsuntersuchung erfolgt. Dies ermöglicht eine vorteilhafte Bilddatenerfassung bei insbesondere Magnetresonanzuntersuchungen, da hierdurch eine Störung und/oder Behinderung der Bilddatenerfassung aufgrund der Displays verhindert werden kann.

Weitere Vorteile, Merkmale und Einzelheiten der Erfindung ergeben sich aus den im Folgenden beschriebenen Ausführungsbeispielen sowie anhand der Zeichnungen.

Es zeigen:
- Fig. 1: eine erfindungsgemäße medizinische Bildgebungsvorrichtung in einer schematischen Darstellung,
- Fig. 2: eine erste Anordnung einer Master-Einheit und mehreren Slave-Einheiten in einer schematische Darstellung,
- Fig. 3: eine zweite Anordnung einer Master-Einheit und mehreren Slave-Einheiten in einer schematische Darstellung und
- Fig. 4: ein erfindungsgemäßes Verfahren zu einem Ansteuern zumindest eines Displays einer medizinischen Bildgebungsvorrichtung.

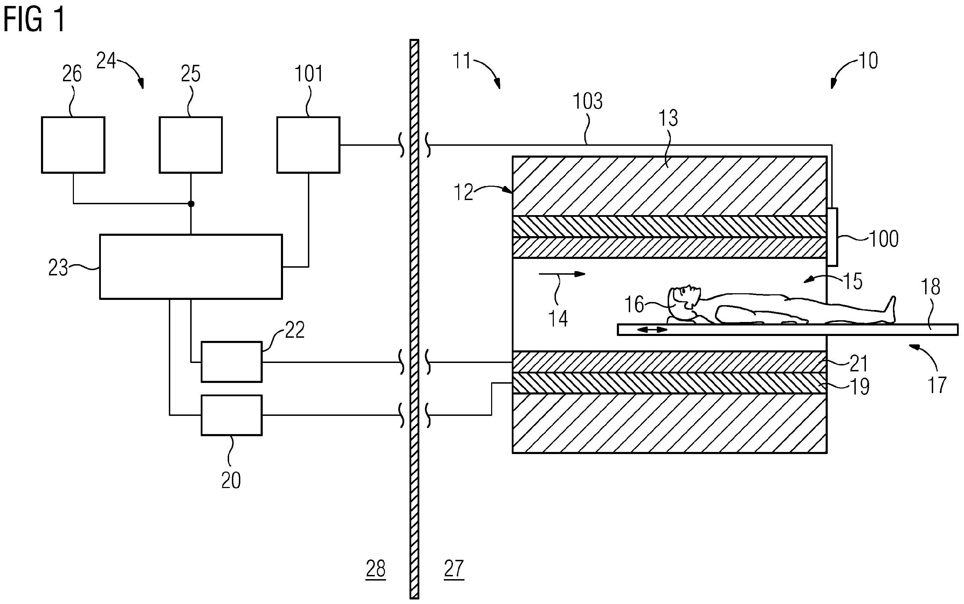

In Fig. 1 ist eine medizinische Bildgebungsvorrichtung 10 schematisch dargestellt. Die medizinische Bildgebungsvorrichtung 10 ist im vorliegenden Ausführungsbeispiel von einer Magnetresonanzvorrichtung 11 gebildet, wobei beispielshaft die vorliegende Erfindung anhand der Magnetresonanzvorrichtung 11 erläutert wird. Die vorliegende Erfindung ist jedoch nicht auf Ausgestaltung der medizinischen Bildgebungsvorrichtung 10 auf eine Magnetresonanzvorrichtung 11 beschränkt und weitere Ausgestaltungen der medizinischen Bildgebungsvorrichtung 10, wie beispielsweise eine Röntgenvorrichtung, eine Computertomographie-Vorrichtung, eine PET-Vorrichtung usw., sind jederzeit denkbar.

Die Magnetresonanzvorrichtung 11 umfasst eine von einer Magneteinheit gebildete Scannereinheit 12, die einen supraleitenden Hauptmagneten 13 zu einem Erzeugen eines starken und insbesondere konstanten Hauptmagnetfelds 14 umfasst. Zudem weist die Magnetresonanzvorrichtung 11 einen Patientenaufnahmebereich 15 auf zu einer Aufnahme eines Patienten 16. Der Patientenaufnahmebereich 15 im vorliegenden Ausführungsbeispiel ist zylinderförmig ausgebildet und in einer Umfangsrichtung von der Magneteinheit zylinderförmig umgeben. Grundsätzlich ist jedoch eine davon abweichende Ausbildung des Patientenaufnahmebereichs 15 jederzeit denkbar. Der Patient 16 kann mittels einer Patientenlagerungsvorrichtung 17 der Magnetresonanzvorrichtung 11 in den Patientenaufnahmebereich 15 geschoben und/oder gefahren werden. Die Patientenlagerungsvorrichtung 17 weist hierzu einen innerhalb des Patientenaufnahmebereichs 15 bewegbar ausgestalteten Patiententisch 18 auf.

Die Scannereinheit 12, insbesondere die Magneteinheit, weist weiterhin eine Gradientenspuleneinheit 19 zu einer Erzeugung von Magnetfeldgradienten auf, die für eine Ortskodierung während einer Bildgebung verwendet werden. Die Gradientenspuleneinheit 19 wird mittels einer Gradientensteuereinheit 20 der Magnetresonanzvorrichtung 11 gesteuert. Die Scannereinheit 12, insbesondere die Magneteinheit, umfasst weiterhin eine Hochfrequenzantenneneinheit 21 zu einer Anregung einer Polarisation, die sich in dem von dem Hauptmagneten 13 erzeugten Hauptmagnetfeld 14 einstellt. Die Hochfrequenzantenneneinheit 21 wird von einer Hochfrequenzantennensteuereinheit 22 der Magnetresonanzvorrichtung 11 gesteuert und strahlt hochfrequente Magnetresonanzsequenzen in den Patientenaufnahmebereich 15 ein.

Zu einer Steuerung des Hauptmagneten 13, der Gradientensteuereinheit 20 und zur Steuerung der Hochfrequenzantennensteuereinheit 22 weist die Magnetresonanzvorrichtung 11 eine Recheneinheit 23 auf. Die Recheneinheit 23 steuert zentral die Magnetresonanzvorrichtung 11, wie beispielsweise das Durchführen einer vorbestimmten bildgebenden Gradientenechosequenz. Zudem umfasst die Recheneinheit 23 eine nicht näher dargestellte Auswerteeinheit zu einer Auswertung von medizinischen Bilddaten, die während der Magnetresonanzuntersuchung erfasst werden.

Zur Steuerung der Magnetresonanzvorrichtung weist die Recheneinheit 23 Computerprogramme, insbesondere Steuerprogramme, und/oder Software auf, die in einer Speichereinheit gespeichert sind. Die Speichereinheit kann dabei von der Recheneinheit 23 umfasst sein oder auch separat zu Recheneinheit 23 ausgebildet sein. Beispielsweise kann die Speichereinheit von der Magnetresonanzvorrichtung 11 umfasst sein oder auch eine externe Speichereinheit umfassen. Die Steuerung der Magnetresonanzvorrichtung erfolgt bei einer Ausführung der Computerprogramme und/oder Software in einem nicht näher dargestellten Prozessor der Recheneinheit 23.

Des Weiteren umfasst die Magnetresonanzvorrichtung 11 eine Benutzerschnittstelle 24, die mit der Recheneinheit 23 verbunden ist. Steuerinformationen wie beispielsweise Bildgebungsparameter, sowie rekonstruierte Magnetresonanzbilder können auf einer Ausgabeeinheit 25, beispielsweise auf zumindest einem Monitor, der Benutzerschnittstelle 24 für ein medizinisches Bedienpersonal angezeigt werden. Weiterhin weist die Benutzerschnittstelle 24 eine Eingabeeinheit 26 auf, mittels der Informationen und/oder Parameter während eines Messvorgangs von dem medizinischen Bedienpersonal eingegeben werden können.

Die Scannereinheit 12 der medizinischen Bildgebungsvorrichtung 10 ist zusammen mit der Patientenlagerungsvorrichtung 17 innerhalb eines Untersuchungsraums 27 angeordnet. Die Recheneinheit 23 mit der Benutzerschnittstelle 24 dagegen ist innerhalb eine Kontrollraums 28 angeordnet. Der Untersuchungsraum 27 und der Kontrollraum 28 sind hierbei getrennt voneinander ausgebildet. Insbesondere ist der Untersuchungsraum 27 hinsichtlich eines Austauschs einer elektromagnetischen Strahlung, insbesondere einer Hochfrequenzstrahlung, von dem Kontrollraum 28 entkoppelt ausgebildet.

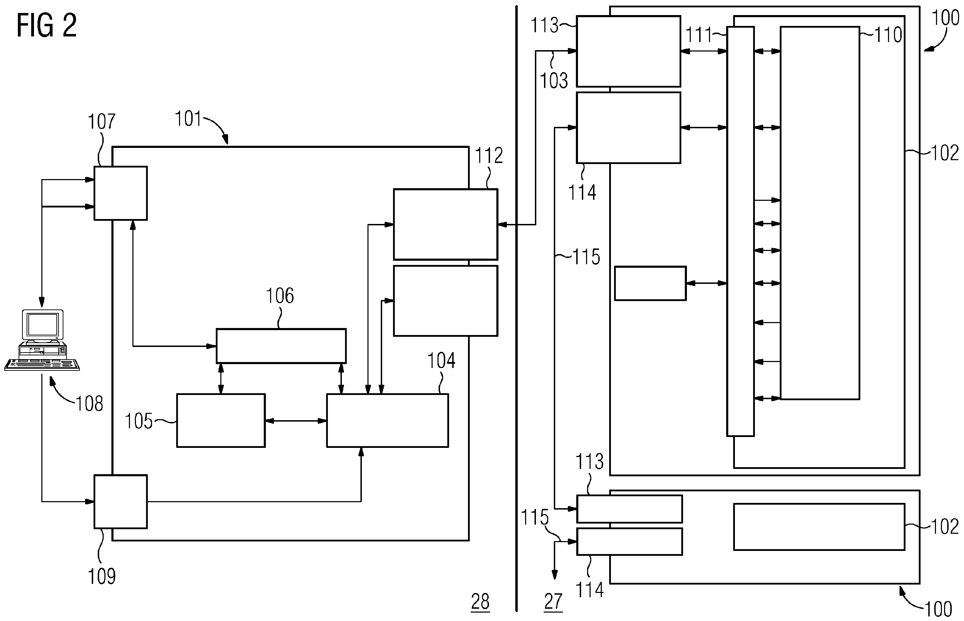

Die medizinische Bildgebungsvorrichtung 10, im vorliegenden Ausführungsbeispiel die Magnetresonanzvorrichtung 11, umfasst weiterhin zumindest ein Display 100, das innerhalb des Untersuchungsraums 27 angeordnet ist. Im vorliegenden Ausführungsbeispiel umfasst die medizinische Bildgebungsvorrichtung 10, insbesondere die Magnetresonanzvorrichtung 11, mehrere Displays 100, die innerhalb des Untersuchungsraums 27 angeordnet sind, wobei in Fig. 2 nur zwei der Displays 100 dargestellt sind. Die Ausgestaltung der medizinischen Bildgebungsvorrichtung 10, insbesondere der Magnetresonanzvorrichtung 11, ist jedoch nicht auf zwei Displays 100 beschränkt. In einer alternativen Ausgestaltung der Erfindung kann die medizinische Bildgebungsvorrichtung 10, insbesondere die Magnetresonanzvorrichtung 11, auch nur ein einziges Display 100 oder auch drei oder mehr Displays 100 umfassen.

Für eine Ansteuerung der einzelnen, im Untersuchungsraum 27 angeordneten Displays 100 weist die medizinische Bildgebungsvorrichtung 10, im vorliegenden Ausführungsbeispiel die Magnetresonanzvorrichtung 11, eine Master-Einheit 101 auf. Die Master-Einheit 101 ist mit der Recheneinheit 23 verbunden. Im vorliegenden Ausführungsbeispiel ist die Master-Einheit 101 separat zur Recheneinheit 23 ausgebildet. Alternativ hierzu kann die Master-Einheit 101 auch von der Recheneinheit 23 umfasst sein oder in die Recheneinheit 23 integriert sein.

Des Weiteren weisen die einzelnen, im Untersuchungsraum 27 angeordneten Displays 100 jeweils eine Slave-Einheit 102 auf. Ein erstes Ausführungsbeispiel für eine Anordnung einer Master-Einheit 101 und mehreren Slave-Einheiten 102 einer medizinischen Bildgebungsvorrichtung 10 ist in Fig. 2 dargestellt.

Die in Fig. 2 dargestellte Master-Einheit 101 ist mittels einer Datenverbindung 103 mit der Slave-Einheit 102 des ersten Displays 100 verbunden. Die Datenverbindung 103 umfasst im vorliegenden Ausführungsbeispiel eine optische Datenverbindung 103 zwischen der Master-Einheit 101 und der Slave-Einheit 102 des ersten Displays 100. Insbesondere umfasst die Datenverbindung 103 eine bidirektionale Datenverbindung 103 zwischen der Master-Einheit 101 und der Slave-Einheit 102. Bevorzugterweise umfasst die optische Datenverbindung 103 Lichtwellenleiter und/oder Glasfaserkabel, die eine Datenübertragung und/oder eine Signalübertragung zwischen der Master-Einheit 101 und der Slave-Einheit 102 des ersten Displays 100 ermöglichen. Dies ist insbesondere bei einer Ausbildung der medizinischen Bildgebungsvorrichtung 10 als Magnetresonanzvorrichtung 11 vorteilhaft, da hier aufgrund von elektrische Leitungen und/oder Kupferleitungen, die elektrischen und/oder elektromagnetischen Felder generieren können, Behinderungen und/oder Störungen bei der Bilddatenerfassung während einer Magnetresonanzuntersuchung auftreten können.

Ist dagegen die medizinische Bildgebungsvorrichtung 10 von einer weiteren, einer Magnetresonanzvorrichtung 11 abweichenden medizinischen Bildgebungsvorrichtung 10 gebildet, kann die Datenverbindung 103 zwischen der Master-Einheit 101 und der zumindest einen Slave-Einheit 102 auch von einer optischen Datenverbindung 103 abweichen. Dabei kann die Datenverbindung 103 eine Ausbildung in einer, dem Fachmann als sinnvoll erscheinender Weise umfassen.

Die Master-Einheit 101 umfasst eine Logikeinheit 104 und/oder eine logische Schaltung, wie beispielsweise ein FPGA, mittels der ein Übertragungsprotokoll, insbesondere ein HMINet-Protokoll, für eine Ansteuerung des zumindest einen Displays 100, insbesondere der mehreren Displays 100, generierbar ist. Mittels der Logikeinheit 104, insbesondere des FPGA, können insbesondere einzelne Zugriffsrechte für die einzelnen Displays 100, insbesondere für die Slave-Einheiten 102 der einzelnen Displays 100, von der Master-Einheit 101 geregelt werden. Bei der Generierung des Übertragungsprotokolls, insbesondere des optischen Übertragungsprotokolls oder eines HMINet-Protokolls, werden insbesondere Standard-Bilddaten und/oder Standard-Bildsignale in das Übertragungsprotokoll, insbesondere das HMINet-Protokoll, gewandelt. Die Standard-Bildsignale können beispielsweise Bildsignale umfassen, die mittels USB, insbesondere USB2 oder USB3, und/oder mittels I²C und/oder mittels Display-Port und/oder LVDS übertragen werden können.

Die Master-Einheit 101 umfasst weiterhin einen Micro-Controller 105 für beispielsweise eine Konfigurierung und/oder Implementierung der Logikeinheit 104. Des Weiteren weist die Master-Einheit 101 ein Ethernet-Modul 106 auf. Das Ethernet-Modul 106 kann beispielsweise eine Ethernet-Verbindung zwischen beispielsweise einem Micro-Controller 105 und der Logikeinheit 104, insbesondere dem FPGA, und zu einer USB-Schnittstelle 107 der Master-Einheit 101 umfassen. Mittels der USB-Schnittstelle 107 kann beispielsweise eine Verbindung zwischen der Master-Einheit 101 und einem zentralen Host-PC 108 der Recheneinheit 23 hergestellt werden. Mittels des Ethernet-Moduls 106 kann eine Ethernet-basierte Diagnose der Master-Einheit 101, beispielsweise der Logikeinheit 104, insbesondere des FPGA, der Master-Einheit 101, durchgeführt werden. Auch kann mittels des Ethernet-Moduls 106 ein Netzwerkmanagement der Master-Einheit 101 oder auch der Datenverbindung 103 mit der Master-Einheit 101 mit den einzelnen Slave-Einheiten 102 erfolgen. Zudem können mittels des Ethernet-Moduls 106 auch Updates für die einzelnen Einheiten der Master-Einheit 101 oder auch Updates für einzelne Einheiten der Displays 100 oder Slave-Einheiten 102 aktualisiert werden.

Die USB-Schnittstelle 107 der Master-Einheit 101 kann dabei eine USB2-Schnittstelle oder auch eine USB3-Schnittstelle umfassen. Zudem umfasst im vorliegenden Ausführungsbeispiel die Master-Einheit 101 auch eine Display-Port-Schnittstelle 109. Der zentrale Host-PC 108 der Recheneinheit 23 ist zusammen mit der Master-Einheit 101 innerhalb des Kontrollraums 28 angeordnet. Der zentrale Host-PC 108 umfasst bevorzugt die Benutzerschnittstelle 24 mit der Eingabeeinheit 26 und der Ausgabeeinheit 27. Zudem ist der zentrale Host-PC 108 mittels der USB-Schnittstelle 107 und mittels der Display-Port-Schnittstelle 109 mit der Master-Einheit 101 verbunden. In einer alternativen Ausgestaltung der Erfindung kann der zentrale Host-PC 108 auch nur mittels der der USB-Schnittstelle 107 oder nur mittels der Display-Port-Schnittstelle 109 mit der Master-Einheit 101 verbunden sein. Zudem sind weitere, dem Fachmann als sinnvoll erscheinenden Schnittstellen für eine Verbindung des zentralen Host-PCs 108 mit der Master-Einheit 101 in einer alternativen Ausgestaltung der Erfindung jederzeit möglich.

Mittels des zentralen Host-PCs 109 und des Ethernet-Moduls 106 kann eine Konfigurierung und/oder Implementierung der Master-Einheit 101 durchgeführt werden. Auch kann mittels des zentralen Host-PCs 109 und des Ethernet-Moduls 106 eine Konfigurierung und/oder Implementierung des Übertragungsprotokolls durchgeführt werden. Des Weiteren kann mittels des zentralen Host-PCs 109 und des Ethernet-Moduls 106 eine Verbindungsdiagnose zwischen der Master-Einheit 101 und den Displays 100, insbesondere den Slave-Einheiten 102, und/oder zwischen den einzelnen Displays 100, insbesondere den einzelnen Slave-Einheiten 102, erfolgen.

Die Slave-Einheiten 102 der einzelnen Displays 100 umfassen jeweils eine eigene Logikeinheit 110. Vorzugsweise umfassen die einzelnen Logikeinheiten 110 jeweils ein FPGA. Mittels der Logikeinheiten 110 kann das von der Master-Einheit 101 an die Displays 100 übertragene Übertragungsprotokoll, insbesondere das HMINet-Protokoll, in den jeweiligen Slave-Einheiten 102 ausgeführt werden. Bei der Ausführung des empfangenen Übertragungsprotokolls, insbesondere des HMINet-Protokolls, können Steuersignale für die einzelnen Displays 100 von den Slave-einheiten 102, insbesondere den Logikeinheiten 110 der Slave-Einheiten 102, generiert werden. Dabei kann das Übertragungsprotokoll, insbesondere das HMINet-Protokoll, in Standard-Bildsignale für Displayschnittstellen der einzelnen Displays 100, wie beispielsweise LVDS und/oder I²C und/oder USB, übertragen werden. Beispielsweise können die die LVDS-Daten und/oder LVDS-Signale für eine Ausgabe und/oder eine Ansteuerung eines LCD-Panels der Displays 100 verwendet werden. Die I²C -Daten und/oder die I²C-Signale können beispielsweise für eine Ausgabe und/oder eine Ansteuerung eines Touch-Controllers der Displays 100 verwendet werden usw. Für eine Übertragung der Standard-Bildsignale von den Slave-Einheiten 102 an Displayschnittstellen weist die Slave-Einheiten jeweils eine Schnittstelle 111 zur Weitergabe der empfangenen Steuersignale und/oder Darstellungsdaten auf.

Die Master-Einheit 101 umfasst weiterhin eine Transceiver-Einheit 112 für eine Übertragung des Übertragungsprotokolls an die Slave-Einheit 102 des ersten Displays 100. Zudem umfasst auch das erste Display 100 eine Transceiver-Einheit 113 auf, mittels der Daten von der Master-Einheit 110 empfangen werden können.

Das erste Display 100 umfasst zudem eine zweite Transceiver-Einheit 114 für eine Verbindung mit dem zweiten Display 100. Hierzu weist auch das zweite Display 100 eine Transceiver-Einheit 113 auf, um Daten, insbesondere das Übertragungsprotokoll, von dem ersten Display 100 zu empfangen. Die Daten, insbesondere das Übertragungsprotokoll, für das zweite Display 100 werden von der Master-Einheit 101 an das erste Display 100 übermittelt und von dort auf das zweite Display 100 übertragen. Hierbei können die Daten für das erste Display 100 zusammen mit den Daten für das zweite Display 100 von der Master-Einheit 101 zunächst auf das erste Display 100 übertragen werden und von dort auf das zweite Display 100 übertragen werden.

Zwischen der Slave-Einheit 102 und/oder Transceiver-Einheit 114 des ersten Displays 100 und der Slave-Einheit 102 und/oder Transceiver-Einheit 113 des zweiten Displays 100 ist eine weitere Datenverbindung 115 der medizinischen Bildgebungsvorrichtung 10 angeordnet. Diese Datenverbindung 115 ist im vorliegenden Ausführungsbeispiel bevorzugt von einer optischen Datenverbindung 115 gebildet. Bevorzugterweise umfasst die optische Datenverbindung 115 Lichtwellenleiter und/oder Glasfaserkabel, die eine Datenübertragung und/oder eine Signalübertragung zwischen der Slave-Einheit 102 des ersten Displays 100 und der Slave-Einheit 102 des zweiten Displays 100 ermöglichen.

Von der Slave-Einheit 102 des ersten Displays 100 werden diejenigen Daten und/oder Anteile des Übertragungsprotokolls, für die die Slave-Einheit 102 des ersten Displays 100 Zugriffsrechte aufweist und/oder Zugriffsrechte von der Master-Einheit 101 zugeteilt bekommen hat, ausgeführt. Die Daten und/oder Anteile des Übertragungsprotokolls, die für das zweite Display 100 oder weitere Displays 100 vorgesehen sind, werden an dem ersten Display 100, insbesondere der Slave-Einheit 102 des ersten Displays 100, für eine Weiterleitung an das zweite Display 100 durchgeschleift. Von der Slave-Einheit 102 des zweiten Displays 100 werden diejenigen Daten und/oder Anteile des Übertragungsprotokolls, für die die Slave-Einheit 102 des zweiten Displays 100 Zugriffsrechte aufweist und/oder Zugriffsrechte von der Master-Einheit 101 zugeteilt bekommen hat, ausgeführt. Die Daten und/oder Anteile des Übertragungsprotokolls, die für weitere Displays 100 vorgesehen sind, werden ebenfalls an dem zweiten Display 100, insbesondere der Slave-Einheit 102 des zweiten Displays 100, für eine Weiterleitung an die weiteren Display 100 durchgeschleift.

Weist beispielsweise die medizinische Bildgebungsvorrichtung 10, insbesondere die Magnetresonanzvorrichtung 11, mehr als zwei Displays 100 auf, so weist auch das zweite Display 100 eine zweite Transceiver-Einheit 114 auf für eine Verbindung mit dem dritten Display 100. Die Daten und/oder Anteile des Übertragungsprotokolls, die für das dritte Display 100 oder weitere Displays 100 vorgesehen sind, werden an dem ersten Display 100, insbesondere der ersten Slave-Einheit 102, und auch an dem zweiten Display 100, insbesondere der zweiten Slave-Einheit 102, für eine Weiterleitung an das dritte Display 100 durchgeschleift. Von der Slave-Einheit 102 des dritten Displays 100 werden diejenigen Daten und/oder Anteile des Übertragungsprotokolls, für die die Slave-Einheit 102 des dritten Displays 100 Zugriffsrechte aufweist und/oder Zugriffsrechte von der Master-Einheit 101 zugeteilt bekommen hat, ausgeführt.

Aufgrund der Datenübertragung an das zweite Display 100 über das erste Display 100 ist somit auch ein Betriebsmodus und/oder Betriebszustand des zweiten Displays 100 und auch von weiteren Displays 100 zumindest teilweise abhängig von einem Betriebsmodus und/oder Betriebszustand des ersten Displays 100. Nur wenn das erste Display 100 sich in einem eingeschalteten Betriebszustand und/oder Betriebsmodus befindet, können Daten an das zweite Display 100 oder die weiteren Displays 100 übertragen werden.

Das zweite Display 100 und das dritte Display 100 oder auch weitere Displays 100 sind analog der obigen Beschreibung ausgebildet, auch wenn in Fig. 2 aufgrund der Übersichtlichkeit von Fig. 2 das zweite Display nur sehr grob skizziert ist und ein drittes Display 100 nur aufgrund der zweiten Transceiver-Einheit 114 des zweiten Displays 100 angedeutet ist.

Allgemeiner formuliert ergibt sich folgender Zusammenhand zwischen den einzelnen Displays 100 der medizinischen Bildgebungsvorrichtung 10:
Umfasst die medizinische Bildgebungsvorrichtung 10 n Displays 100, wobei n≥2 ist, umfasst dabei das (n-1)-te Display 100 eine Transceiver-Einheit 114 für eine Datenverbindung mit dem n-ten Display 100. Dabei umfasst jedes (n-1)-te Display 100 eine erste Transceiver-Einheit 113 für einen Eingang und/oder Empfang des Übertragungsprotokolls und zweite Transceiver-Einheit 114 für ein Weiterleiten des Übertragungsprotokolls an das n-te Display 100. Hierbei ist auch ein Betriebsmodus des n-ten Displays 100 zumindest teilweise abhängig von einem Betriebsmodus den (n-1)-ten Displays 100. Im Ausführungsbeispiel der Fig. 2 ist n>2.

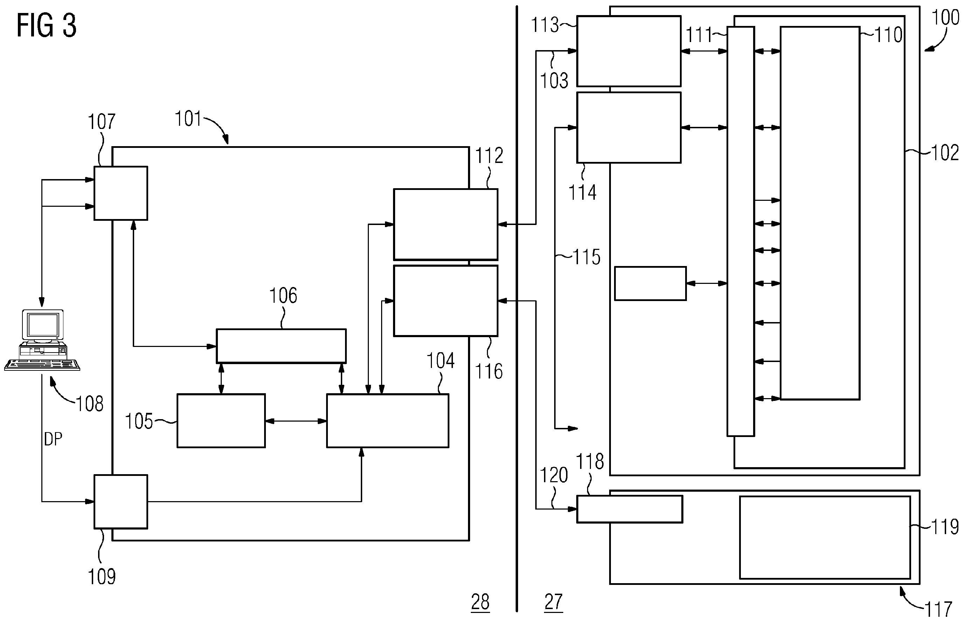

In Fig. 3 ist ein alternatives Ausführungsbeispiel der Master-Einheit 101 dargestellt. Im Wesentlichen gleich bleibende Bauteile, Merkmale und Funktionen sind grundsätzlich mit den gleichen Bezugszeichen beziffert. Die nachfolgende Beschreibung beschränkt sich im Wesentlichen auf die Unterschiede zu dem Ausführungsbeispiel in Fig. 2, wobei bezüglich gleich bleibender Bauteile, Merkmale und Funktionen auf die Beschreibung des Ausführungsbeispiels in Fig. 2 verwiesen wird.

Die Master-Einheit 101 weist in Fig. 3 zwei Transceiver-Einheiten 112, 116 auf. Die erste Transceiver-Einheit 112 ist für eine Übertragung des Übertragungsprotokolls an die Slave-Einheit 102 des ersten Displays 100 vorgesehen. Die zweite Transceiver-Einheit 116 ist für eine Übertragung eines Übertragungsprotokolls an eine weitere Einheit 117, die im vorliegenden Ausführungsbeispiel eine USB-Schnittstelle umfasst, vorgesehen. Bevorzugt umfasst die USB-Schnittstelle eine USB3-Schnittstelle für eine Anbindung eines USB-Geräts, insbesondere eines Geräts mit einer USB-Schnittstelle. Alternativ hierzu kann die weitere Einheit 117 auch ein weiteres Display umfassen.

Die weitere Einheit 117 weist im vorliegenden Ausführungsbeispiel ebenfalls eine Transceiver-Einheit 118 und eine Slave-Einheit 19 auf. Die weitere Einheit 117 ist ebenfalls über eine Datenverbindung 120 mit der Master-Einheit 101 verbunden. Dabei ist die Datenverbindung 120 zwischen der Transceiver-Einheit 116 der Master-Einheit 101 und der Transceiver-Einheit 118 der weiteren Einheit 117 angeordnet. Die Datenverbindung 120 ist im vorliegenden Ausführungsbeispiel bevorzugt von einer optischen Datenverbindung 120 gebildet. Bevorzugterweise umfasst die optische Datenverbindung 120 Lichtwellenleiter und/oder Glasfaserkabel, die eine Datenübertragung und/oder eine Signalübertragung ermöglichen.

Eine Anordnung der mehreren Displays 100, die über die Datenverbindung 103 mit der ersten Transceiver-Einheit 112 der Master-Einheit 101 verbunden sind, ist analog zur Beschreibung zu Fig. 2 ausgebildet, auch wenn in Fig. 3 dies nur durch Darstellung von einem Display 100 angedeutet ist.

Die dargestellte medizinische Bildgebungsvorrichtung 10, insbesondere die Magnetresonanzvorrichtung 11, kann selbstverständlich weitere Komponenten umfassen, die medizinische Bildgebungsvorrichtungen 10, insbesondere die Magnetresonanzvorrichtungen 11, gewöhnlich aufweisen. Eine allgemeine Funktionsweise einer medizinischen Bildgebungsvorrichtung 10, insbesondere der Magnetresonanzvorrichtung 11, ist zudem dem Fachmann bekannt, so dass auf eine detaillierte Beschreibung der weiteren Komponenten verzichtet wird.

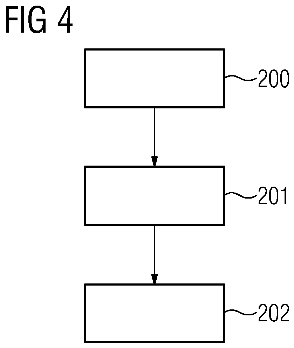

In Fig. 4 ist ein erfindungsgemäßes Verfahren zu einem Ansteuern zumindest eines Displays 100 der medizinischen Bildgebungsvorrichtung 10 dargestellt. Die medizinische Bildgebungsvorrichtung 10, insbesondere Magnetresonanzvorrichtung 11, ist gemäß den oben dargestellten Ausführungen der Fig. 1 bis 3 ausgebildet.

In einem ersten Verfahrensschritt 200 des erfindungsgemäßen Verfahrens erfolgt ein Bereitstellen eines Übertragungsprotokolls innerhalb der Master-Einheit 101 der medizinischen Bildgebungsvorrichtung 10. In einem zweiten Verfahrensschritt 201 erfolgt dann ein Übertragen des Übertragungsprotokolls an die Slave-Einheit 102 des ersten Displays 100 mittels der Datenverbindung 103. In einem dritten Verfahrensschritt 202 erfolgt ein Ausführen des Übertragungsprotokolls in der Slave-Einheit 102 des ersten Displays 100.

Weist das Übertragungsprotokoll Daten für das erste Display 100 und für weitere Displays 100 auf, werden die Daten für die weiteren Displays 100 von der Master-Einheit 101 an das erste Display 100, insbesondere an die Slave-Einheit 102 des ersten Displays 100, und von dort an die weiteren Display 100, insbesondere an die Slave-Einheiten 102 der weiteren Displays 100, in dem zweiten Verfahrensschritt 201 übertragen. Die Daten und/oder Anteile des Übertragungsprotokolls, die für weitere Displays 100 vorgesehen sind, werden in diesem Verfahrensschritt 201 in dem ersten Display 100, insbesondere der Slave-einheit 102 des ersten Displays 100, für eine Weiterleitung an die weiteren Display 100 durchgeschleift.

Das bereitgestellte Übertragungsprotokoll kann zudem aufgrund des Ethernet-Moduls 106 der Master-Einheit 101 für eine Ethernet-basierte Auswertung der Verbindung zwischen der Master-Einheit 101 und den Slave-Einheiten 102 oder auch zwischen den einzelnen Slave-Einheiten 102 durchgeführt werden. Zudem kann auch ein Ethernet-basiertes Netzwerkmanagement durchgeführt werden.

Mittels der Recheneinheit 23 der medizinischen Bildgebungsvorrichtung 10, insbesondere der Magnetresonanzvorrichtung 11, kann zudem ein automatisches Abschalten der einzelnen Displays 100 erfolgen. Bevorzugt erfolgt das automatische Abschalten von einzelnen Displays 100 während der medizinischen Bildgebungsuntersuchung, insbesondere während der Magnetresonanzuntersuchung.

Obwohl die Erfindung im Detail durch das bevorzugte Ausführungsbeispiel näher illustriert und beschrieben wurde, so ist die Erfindung nicht durch die offenbarten Beispiele eingeschränkt und andere Variationen können vom Fachmann hieraus abgeleitet werden, ohne den Schutzumfang der Erfindung zu verlassen.

## Patentansprüche

1. Medizinische Bildgebungsvorrichtung mit einer medizinischen Scannereinheit, einer Recheneinheit, einer Master-Einheit, die mit der Recheneinheit verbunden ist, und zumindest einem Display,
**dadurch gekennzeichnet, dass** das zumindest eine Display eine Slave-Einheit umfasst, wobei die Master-Einheit mittels einer Datenverbindung der medizinischen Bildgebungsvorrichtung mit der Slave-Einheit verbunden ist.

2. Medizinische Bildgebungsvorrichtung nach Anspruch 1,
**dadurch gekennzeichnet, dass** die Datenverbindung eine optische Datenverbindung zwischen der Slave-Einheit und der Master-Einheit umfasst.

3. Medizinische Bildgebungsvorrichtung nach Anspruch 2,
**dadurch gekennzeichnet, dass** die Datenverbindung eine bidirektionale Datenverbindung umfasst.

4. Medizinische Bildgebungsvorrichtung nach einem der vorhergehenden Ansprüche,
**dadurch gekennzeichnet, dass** die Master-Einheit eine Logikeinheit aufweist, mittels der ein Übertragungsprotokoll generierbar ist.

5. Medizinische Bildgebungsvorrichtung nach einem der vorhergehenden Ansprüche,
**dadurch gekennzeichnet, dass** die zumindest eine Slave-Einheit eine Logikeinheit umfasst, mittels der ein Übertragungsprotokoll ausführbar ist.

6. Medizinische Bildgebungsvorrichtung nach einem der vorhergehenden Ansprüche,
**dadurch gekennzeichnet, dass** die Slave-Einheit des zumindest einen Displays eine Schnittstelle zur Weitergabe der empfangenen Steuersignale und/oder Darstellungsdaten umfasst.

7. Medizinische Bildgebungsvorrichtung nach einem der vorhergehenden Ansprüche,
**dadurch gekennzeichnet, dass** das Display einen Transceiver-Einheit und die Master-Einheit eine Transceiver-Einheit aufweisen.

8. Medizinische Bildgebungsvorrichtung nach einem der vorhergehenden Ansprüche,
**dadurch gekennzeichnet, dass** das Display von einem ersten Display gebildet ist, wobei die medizinisches Bildgebungsvorrichtung zumindest ein weiteres Display mit einer Slave-Einheit umfasst.

9. Medizinische Bildgebungsvorrichtung nach einem der vorhergehenden Ansprüche,
**gekennzeichnet durch** n Displays mit n≥2, wobei das (n-1)-te Display eine Transceiver-Einheit für eine Datenverbindung mit dem n-ten Display aufweist.

10. Medizinische Bildgebungsvorrichtung nach Anspruch 9,
**dadurch gekennzeichnet, dass** jedes (n-1)-te Display eine erste Transceiver-Einheit für einen Eingang des Übertragungsprotokolls und eine zweite Transceiver-Einheit für ein Weiterleiten des Übertragungsprotokolls aufweist.

11. Medizinische Bildgebungsvorrichtung nach einem der Ansprüche 9 bis 10,
**dadurch gekennzeichnet, dass** ein Betriebsmodus des n-ten Displays zumindest teilweise abhängig ist von einem Betriebsmodus des (n-1)-ten Displays.

12. Medizinische Bildgebungsvorrichtung nach einem der vorhergehenden Ansprüche,
**dadurch gekennzeichnet, dass** die Master-Einheit eine erste Transceiver-Einheit umfasst für eine Übertragung eines Übertragungsprotokolls an das zumindest eine Display und eine zweite Transceiver-Einheit für eine Übertragung eines weiteren Übertragungsprotokolls an eine weitere Einheit, die eine Slave-Einheit umfasst.

13. Medizinische Bildgebungsvorrichtung nach einem der vorhergehenden Ansprüche,
**dadurch gekennzeichnet, dass** die Master-Einheit ein Ethernet-Modul umfasst.

14. Medizinische Bildgebungsvorrichtung nach einem der vorhergehenden Ansprüche,
**gekennzeichnet, durch** einen zentralen Host-PC, der zusammen mit der Master-Einheit innerhalb eines Kontrollraums angeordnet ist.

15. Medizinische Bildgebungsvorrichtung nach Anspruch 14,
**dadurch gekennzeichnet, dass** die Master-Einheit zumindest eine standardisierte Datenschnittstelle und/oder Grafikschnittstelle aufweist zu einem Datenaustausch mit dem zentralen Host-PC.

16. Verfahren zu einem Ansteuern zumindest eines Displays einer medizinischen Bildgebungsvorrichtung, die nach einem der Ansprüche 1 bis 14 ausgebildet ist, mit den folgenden Schritten:
- Bereitstellen eines Übertragungsprotokolls innerhalb einer Master-Einheit,
- Übertragen des Übertragungsprotokolls mittels einer Datenverbindung an eine Slave-Einheit des zumindest einen Displays und
- Ausführen des Übertragungsprotokolls in der Slave-Einheit.

17. Verfahren nach Anspruch 16,
**dadurch gekennzeichnet, dass** das Übertragungsprotokoll Daten für ein erstes Display und Daten für zumindest ein weiteres Display umfasst, wobei die Daten für das zumindest eine weitere Display von der Master-Einheit an die Slave-Einheit des ersten Displays übertragen werden und von der Slave-Einheit des ersten Displays an eine Slave-Einheit des zumindest einen weiteren Displays übertragen werden.

18. Verfahren nach einem der Ansprüche 16 bis 17,
**dadurch gekennzeichnet, dass** eine Ethernet-basierte Auswertung einer Verbindung zwischen der Master-Einheit und dem zumindest einen Display und/oder ein Ethernet-basiertes Netzwerkmanagement durchgeführt wird.

19. Verfahren nach einem der Ansprüche 16 bis 18,
**dadurch gekennzeichnet, dass** ein automatisches Abschalten des zumindest einen Displays während einer medizinischen Bildgebungsuntersuchung erfolgt.
